Europäisches Patentamt

European Patent Office

Office européen des brevets

(19)

(11) Veröffentlichungsnummer: **0 495 340 A1**

# EUROPÄISCHE PATENTANMELDUNG

(12)

(21) Anmeldenummer: **91810972.9**

(22) Anmeldetag: **13.12.91**

(51) Int. Cl.5: **A61F 2/38**

(30) Priorität: **18.01.91 CH 143/91**

(43) Veröffentlichungstag der Anmeldung:
**22.07.92 Patentblatt 92/30**

(84) Benannte Vertragsstaaten:
**AT CH DE FR GB IT LI**

(71) Anmelder: **GEBRÜDER SULZER
AKTIENGESELLSCHAFT
Zürcherstrasse 12
CH-8401 Winterthur(CH)**

(72) Erfinder: **Moser, Walter
Stuckishausstrasse 19
CH-3037 Herrenschwanden(CH)**
Erfinder: **Willi, Roland
Winterthurerstrasse 90
CH-8413 Neftenbach(CH)**

(54) **Modularer Bausatz für den Tibiateil einer Kniegelenkprothese.**

(57) In den Figuren ist ein modularer Bausatz für den Tibiateil einer Kniegelenkprothese gezeigt, der aus einem Tibiaplateau (1) mit Zapfen (2), aus einem in die Tibia hineinragenden Stamm (3) und aus einer Gelenkauflage (4) aus Polyäthylen besteht. In das Tibiaplateau (1) sind unterschiedlich grosse Stämme (3) einsetzbar und unterschiedlich hohe Gelenkauflagen (4) sind in einer Ebene des Tibiaplateaus allseitig geführt und durch eine Schwenkbewegung in diese Ebene einbringbar. Dies geschieht, indem Vorsprünge (7) der Gelenkauflagen (4) in einer tangential angeordneten Schwenkachse (5) in Aussparungen des Tibiaplateaus eingreifen und indem entgegengesetzt zur Schwenkachse eine Schnappverbindung (9) zwischen Gelenkauflage (4) und Tibiaplateau (1) einrastet.

Fig.1

EP 0 495 340 A1

Die Erfindung betrifft einen modularen Bausatz für den Tibiateil einer Kniegelenkprothese bestehend aus einem Tibiaplateau mit Zapfen aus einem in die Tibia hineinragenden Stamm und aus einer Gelenkauflage aus Polyäthylen.

In der CH-PS 667 383 ist der Tibiateil einer Kniegelenkprothese beschrieben, die aus einem Tibiaplateau mit Zapfen und aus einer Gelenkauflage besteht. Der Einbau dieser Implantate verlangt mehrere individuelle Vorbearbeitungen bei der Resektion des Tibiaknochens, die vom mechanischen Geschick des Operateurs abhängen, um das Implantat auf der ganzen vorgesehenen Fläche abzustützen.

Aufgabe der Erfindung ist es, dem Operateur, der entsprechend dem vorgefundenen Knochenzustand eine einfache Resektion vorgenommen hat, ein Implantat zur Verfügung zu stellen, dessen Befestigungs- und Einbaulänge intraoperativ einstellbar sind. Diese Aufgabe wird mit der Erfindung dadurch gelöst, dass in das Tibiaplateau unterschiedlich grosse Stämme einsetzbar sind und dass unterschiedlich hohe Gelenkauflagen in einer Ebene des Tibiaplateaus allseitig geführt durch eine Schwenkbewegung in diese Ebene einbringbar sind, indem Vorsprünge der Gelenkauflagen in einer tangential angeordneten Schwenkachse in Aussparungen des Tibiaplateaus eingreifen und indem entgegengesetzt zur Schwenkachse eine Schnappverbindung zwischen Gelenkauflage und Tibiaplateau einrastet.

Der Vorteil der Erfindung besteht darin, dass mit einem ebenen, in der richtigen Winkellage zur Tibia durchgeführten Resektionsschnitt und mit der Festlegung des Bohrbildes in der Resektionsfläche alle weiteren Funktionsmasse durch Zusammenfügen von vorgefertigten Bauteilen gebildet werden. Die abhängigen Ansprüche 2 und 3 beziehen sich auf vorteilhafte Weiterbildungen der Erfindung.

Im folgenden wird die Erfindung anhand eines Ausführungsbeispiels im Zusammenhang mit der Zeichnung näher erläutert. Es zeigen:

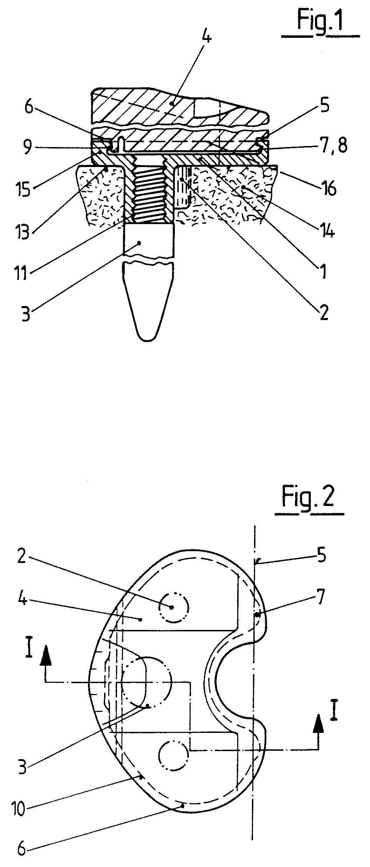

Fig. 1    schematisch einen abgesetzten Längsschnitt (I-I, Fig. 2) durch den Tibiateil für eine Kniegelenkprothese;

Fig. 2    schematisch die Draufsicht auf eine Gelenkauflage gemäss Fig. 1, unter der die Lage von Zapfen, Stamm und Schwenkachse angedeutet ist; und

Fig. 3    schematisch den vergrösserten Ausschnitt einer Schnappverbindung gemäss Fig. 1.

In den Figuren ist ein modularer Bausatz für den Tibiateil einer Kniegelenkprothese gezeigt, der aus einem Tibiaplateau mit Zapfen, aus einem in die Tibia hineinragenden Stamm und aus einer Gelenkauflage aus Polyäthylen besteht.

In das Tibiaplateau sind unterschiedlich grosse Stämme einsetzbar und unterschiedlich hohe Gelenkauflagen sind in einer Ebene des Tibiaplateaus allseitig geführt und durch eine Schwenkbewegung in diese Ebene einbringbar. Dies geschieht, indem Vorsprünge der Gelenkauflagen in einer tangential angeordneten Schwenkachse in Aussparungen des Tibiaplateaus eingreifen und indem entgegengesetzt zur Schwenkachse eine Schnappverbindung zwischen Gelenkauflage und Tibiaplateau einrastet.

Ein Tibiaplateau 1 besitzt ein festes Bohrbild für zwei Zapfen 2 und einen dazwischen liegenden Stamm 3, welches auf die ebene Resektionsfläche 13 eines Tibiaknochens 14 übertragen wird. Die Bohrungen für die Zapfen 2 und die Zapfendimensionen bleiben unverändert, da die Zapfen hauptsächlich der Verdrehsicherung in der Resektionsebene dienen, während der auswechselbare Stamm 3 und seine zugehörige Bohrung in ihrer Grösse stufenweise den Befestigungsmöglichkeiten im Tibiaknochen anpassbar sind, um das Tibiaplateau zu verankern. Die unterschiedlichen Zapfen 3 sind mit einer Verschraubung 11 im Tibiaplateau 1 befestigt.

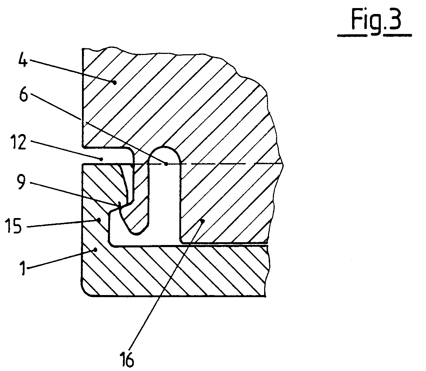

Die Gelenkauflagen 4 liegen in unterschiedliche Höhen gestuft vor, während die Anschlussmasse zu einem Tibiaplateau 1 für alle Höhen gleich sind. Die Gelenkauflagen 4 liegen in einer Ebene 6, die durch den hochgezogenen Begrenzungsrand 15 des Tibiaplateaus gebildet wird, auf und sind in dieser Ebene durch Führungselemente 10 mit ihrem vorstehenden Innenteil 16 geführt, das der Kontur des hochstehenden Begrenzungsrandes 15 nachgeformt ist. Posterior weist der Begrenzungsrand 15 auf seiner Innenseite zwei Aussparungen 8 auf, die auf einer gemeinsamen Tangente an den Innenteil 16 liegen, während der Innenteil 16 der Vorsprünge 7 besitzt, die durch eine Schwenkbewegung in die Aussparungen 8 einhängbar sind. Die Vorsprünge 7 und die Aussparungen 8 bilden eine Schwenkachse 5, an der durch Einschwenken der Gelenkauflage 4 eine einseitige Sicherung gegen Herausfallen aus der Ebene 6 entsteht. Auf der Gegenseite zur Schwenkbachse 5 ist eine Schappverbindung 9 zwischen Begrenzungsrand 15 und Innenteil 16 angebracht, die mit dem Aufliegen der Gelenkauflage 4 auf der Ebene 6 einrastet. In Fig. 3 ist über der Schnappverbindung 9 zwischen Begrenzungsrand 15 und Gelenkauflage 4 eine Ausnehmung 12 gezeigt, in die ein schraubenzieherähnliches Werkzeug als Hebel einsetzbar ist, um die Verhakung in der Schnappverbindung 9 zu lösen. Die Gelenkauflage 4 ist unabhängig von der Schnappverbindung senkrecht zur Schwenkachse 5 in der Ebene 6 durch den Begrenzungsrand 15 geführt, damit keine Verschiebekräfte in der Ebene 6 auf die Schnappverbindung 9 wirken können, d.h. die Schnappverbindung 9 sichert senkrecht zur Ebene 6 und wird nicht durch wechselnde Zentrierkräfte ermüdet. Im montierten Zustand sind der Innenteil 16 und der Grund des Tibiaplateaus 1 weniger als 1/10 mm voneinander entfernt, damit im Rahmen der Elastizität der beiden Teile weitere tragende Flächen bei Belastung entstehen.

plateau (1) übertragen.

## Interne Stückliste

| | |
|---|---|
| 1 | Tibiaplateau |
| 2 | Zapfen |
| 3 | Stamm |
| 4 | Gelenkauflage |
| 5 | Schwenkachse |
| 6 | Ebene |
| 7 | Vorsprung |
| 8 | Aussparung |
| 9 | Schnappverbindung |
| 10 | Führungselemente |
| 11 | Verschraubung |
| 12 | Ausnehmung |
| 13 | Resektionsfläche |
| 14 | Tibiaknochen |
| 15 | Begrenzungsrand |
| 16 | Innenteil |

## Patentansprüche

1.  Modularer Bausatz für den Tibiateil einer Knie-gelenkprothese bestehend aus einem Tibiapla-teau mit Zapfen aus einem in die Tibia hineinragenden Stamm und aus einer Gelenkauflage aus Polyäthylen, **dadurch gekennzeichnet,** dass in das Tibiaplateau (1) unterschiedlich grosse Stämme (3) einsetzbar sind und dass unterschiedlich hohe Gelenkauflagen (4) in einer Ebene (6) des Tibiaplateaus (1) allseitig geführt durch eine Schwenkbewegung in diese Ebene (6) einbringbar sind, indem Vorsprünge (7) der Gelenkauflagen (4) in einer tangential angeordneten Schwenkachse (5) in Aussparungen (8) des Tibiaplateaus (1) eingreifen und indem entgegengesetzt zur Schwenkachse (5) eine Schnappverbindung (9) zwischen Gelenkauflage (4) und Tibiaplateau (1) einrastet.

2.  Modularer Bausatz nach Anspruch 1, dadurch gekennzeichnet, dass die Schwenkachse (5) zum Einhängen der Vorsprünge (7) posterior und die Schnappverbindung (9) anterior angeordnet ist.

3.  Modularer Bausatz nach Anspruch 1, dadurch gekennzeichnet, dass Führungselemente (10) unabhängig von der Schnappverbindung (9) in der Ebene (6), die durch das Einschwenken in die Endlage definiert ist, so angeordnet sind, dass sie beliebig in dieser Ebene (6) orientierte Kräfte zwischen Gelenkauflage (4) und Tibia-

_Fig.1_

_Fig.2_

Fig.3

| | Europäisches Patentamt | EUROPÄISCHER RECHERCHENBERICHT | Nummer der Anmeldung |
|---|---|---|---|

EP    91 81 0972

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 944 757 (MARTINEZ ET AL.)<br>* Spalte 2, Zeile 62 - Zeile 65 *<br>* Spalte 4, Zeile 37 - Zeile 45 *<br>* Spalte 5, Zeile 19 - Zeile 27 *<br>--- | 1,2 | A61F2/38 |
| Y | US-A-4 795 468 (HODOREK ET AL.)<br>* Spalte 5, Zeile 68 - Spalte 6, Zeile 18;<br>Abbildungen *<br>--- | 1,2 | |
| A | EP-A-0 246 050 (DOW CORNING CORP.)<br>* Spalte 6, Zeile 24 - Zeile 48; Abbildung 6 *<br>--- | 3 | |
| X,P | FR-A-2 653 992 (BERAKASSA ET AL.)<br>* Ansprüche 1,2; Abbildungen *<br><br>----- | 1 | |
| | | | RECHERCHIERTE SACHGEBIETE (Int. Cl.5)<br><br>A61F |

Der vorliegende Recherchenbericht wurde für alle Patentansprüche erstellt

| Recherchenort | Abschlußdatum der Recherche | Prüfer |
|---|---|---|
| DEN HAAG | 07 APRIL 1992 | VILLENEUVE J.M. |